# EUROPEAN PATENT APPLICATION

(11) **EP 1 182 253 A2**
(43) Date of publication of application: **27.02.2002**
(21) Application number: 01115642.9
(22) Date of filing: 03.07.2001
(51) Int. Cl.: C12N 15/00, C12N 15/53, C12N 9/04, C12N 5/00

(54) **Method for improving the thermostability of proteins**

(30) Priority: 04.07.2000 JP 2000201920; 31.05.2001 JP 2001164332
(71) Applicant: Ajinomoto Co., Inc., Tokyo 104 (JP)
(72) Inventor: Yamagishi, Akihiko, Itabashi-ku, Tokyo (JP)
(74) Representative: HOFFMANN - EITLE

(57) **Abstract**

The present invention provides a method for improving thermostability of proteins, proteins having improved thermostability, nucleic acids encoding the proteins and host cells producing the proteins improved in thermostability.

The method for improving thermostability of protein comprises:
(i) comparing amino acid sequences of proteins derived from two or more species which evolutionarily correspond to each other in a phylogenetic tree,
(ii) estimating an amino acid sequence of an ancestral protein corresponding to the amino acid sequences compared in step (i),
(iii) and comparing the amino acid residues in the amino acid sequence in one of the proteins compared in step (i) with amino acid residues at a corresponding position in the ancestral protein estimated in step (ii), and replacing one or more of the amino acid residues different from those of the ancestral protein with the same amino acid residues as those of the ancestral protein.

SEQUENCE LISTING

<110> Ajinomoto Co., Inc.

<120> A method for improving the thermostability of a
      protein, a protein having improved thermostability and
      a nucleic acid sequence encoding the protein

<130> OP01088

<140>
<141>

<150> JP 2000-201920
<151> 2000-07-04

<150> JP 2001-164332
<151> 2001-05-31

<160> 104

<170> PatentIn Ver. 2.1

<210> 1
<211> 9
<212> PRT
<213> Sulfolobus sp.

<400> 1

Tyr Asp Met Tyr Ala Asn Ile Arg Pro
1               5

5   <210> 2
<211> 9
<212> PRT
<213> Sulfolobus sp.

10   <400> 2
Ile Ala Lys Val Gly Leu Asn Phe Ala
1               5

15   <210> 3
<211> 8
<212> PRT
<213> Sulfolobus sp.

20   <400> 3
Val His Gly Ala Ala Phe Asp Ile
1               5

25   <210> 4
<211> 6
<212> PRT
<213> Sulfolobus sp.

30   <400> 4

Met Met Tyr Glu Arg Met
1               5

<210> 5
<211> 9
<212> PRT
<213> Thermus thermophilus

<400> 5
Gln Asp Leu Phe Ala Asn Leu Arg Pro
1               5

<210> 6
<211> 9
<212> PRT
<213> Thermus thermophilus

<400> 6
Val Ala Arg Val Ala Phe Glu Ala Ala
1               5

<210> 7
<211> 8
<212> PRT
<213> Thermus thermophilus

<400> 7

Val His Gly Ser Ala Pro Asp Ile
1               5

<210> 8
<211> 6
<212> PRT
<213> Thermus thermophilus

<400> 8
Met Met Leu Glu His Ala
1               5

<210> 9
<211> 9
<212> PRT
<213> Bacillus subtilis

<400> 9
Leu Asp Leu Phe Ala Asn Leu Arg Pro
1               5

<210> 10
<211> 9
<212> PRT
<213> Bacillus subtilis

<400> 10

Val Ile Arg Glu Gly Phe Lys Met Ala
1               5

<210> 11
<211> 8
<212> PRT
<213> Bacillus subtilis

<400> 11
Val His Gly Ser Ala Pro Asp Ile
1               5

<210> 12
<211> 6
<212> PRT
<213> Bacillus subtilis

<400> 12
Met Leu Leu Arg Thr Ser
1               5

<210> 13
<211> 9
<212> PRT
<213> Escherichia coli

<400> 13

Phe Lys Leu Phe Ser Asn Leu Arg Pro
1               5

<210> 14
<211> 9
<212> PRT
<213> Escherichia coli

<400> 14
Ile Ala Arg Ile Ala Phe Glu Ser Ala
1               5

<210> 15
<211> 8
<212> PRT
<213> Escherichia coli

<400> 15
Ala Gly Gly Ser Ala Pro Asp Ile
1               5

<210> 16
<211> 6
<212> PRT
<213> Escherichia coli

<400> 16

Leu Leu Leu Arg Tyr Ser
1               5

<210> 17
<211> 9
<212> PRT
<213> Agrobacterium tumefaciens

<400> 17
Leu Glu Leu Phe Ala Asn Leu Arg Pro
1               5

<210> 18
<211> 9
<212> PRT
<213> Agrobacterium tumefaciens

<400> 18
Ile Ala Ser Val Ala Phe Glu Leu Ala
1               5

<210> 19
<211> 8
<212> PRT
<213> Agrobacterium tumefaciens

<400> 19

Val His Gly Ser Ala Pro Asp Ile
1               5

<210> 20
<211> 6
<212> PRT
<213> Agrobacterium tumefaciens

<400> 20
Met Cys Leu Arg Tyr Ser
1               5

<210> 21
<211> 9
<212> PRT
<213> Saccharomyces cerevisiae

<400> 21
Leu Gln Leu Tyr Ala Asn Leu Arg Pro
1               5

<210> 22
<211> 9
<212> PRT
<213> Saccharomyces cerevisiae

<400> 22

Ile Thr Arg Met Ala Ala Phe Met Ala
1               5

<210> 23
<211> 8
<212> PRT
<213> Saccharomyces cerevisiae

<400> 23
Cys His Gly Ser Ala Pro Asp Leu
1               5

<210> 24
<211> 6
<212> PRT
<213> Saccharomyces cerevisiae

<400> 24
Met Met Leu Lys Leu Ser
1               5

<210> 25
<211> 9
<212> PRT
<213> Neurospora crassa

<400> 25

Leu Gly Thr Tyr Gly Asn Leu Arg Pro
1               5

<210> 26
<211> 9
<212> PRT
<213> Neurospora crassa

<400> 26
Ile Ala Arg Leu Ala Gly Phe Leu Ala
1               5

<210> 27
<211> 8
<212> PRT
<213> Neurospora crassa

<400> 27
Ile His Gly Ser Ala Pro Asp Ile
1               5

<210> 28
<211> 6
<212> PRT
<213> Neurospora crassa

<400> 28

Met Met Leu Arg Tyr Ser
1               5

<210> 29
<211> 9
<212> PRT
<213> Saccharomyces cerevisiae

<400> 29
Phe Gly Leu Phe Ala Asn Val Arg Pro
1               5

<210> 30
<211> 9
<212> PRT
<213> Bos taurus

<400> 30
Val Ile Arg Tyr Ala Phe Glu Tyr Ala
1               5

<210> 31
<211> 8
<212> PRT
<213> Saccharomyces cerevisiae

<400> 31

Val His Gly Ser Ala Pro Asp Ile
1               5

<210> 32
<211> 6
<212> PRT
<213> Saccharomyces cerevisiae

<400> 32
Met Met Leu Asn His Met
1               5

<210> 33
<211> 9
<212> PRT
<213> Bos taurus

<400> 33
Phe Asp Leu Tyr Ala Asn Val Arg Pro
1               5

<210> 34
<211> 9
<212> PRT
<213> Bos taurus

<400> 34

Ile Ala Glu Phe Ala Phe Glu Tyr Ala
1               5

<210> 35
<211> 8
<212> PRT
<213> Bos taurus

<400> 35
Val His Gly Ser Ala Pro Asp Ile
1               5

<210> 36
<211> 6
<212> PRT
<213> Bos taurus

<400> 36
Met Met Leu Arg His Met
1               5

<210> 37
<211> 9
<212> PRT
<213> Bacillus subtilis

<400> 37

Leu Asp Leu Phe Val Cys Leu Arg Pro
1               5

<210> 38
<211> 9
<212> PRT
<213> Bacillus subtilis

<400> 38
Leu Val Arg Ala Ala Ile Asp Tyr Ala
1               5

<210> 39
<211> 8
<212> PRT
<213> Bacillus subtilis

<400> 39
Thr His Gly Thr Ala Pro Lys Tyr
1               5

<210> 40
<211> 6
<212> PRT
<213> Bacillus subtilis

<400> 40

Leu Leu Leu Glu His Leu
1               5

<210> 41
<211> 9
<212> PRT
<213> Escherichia coli

<400> 41
Leu Asp Leu Tyr Ile Cys Leu Arg Pro
1               5

<210> 42
<211> 9
<212> PRT
<213> Escherichia coli

<400> 42
Leu Val Arg Ala Ala Ile Glu Tyr Ala
1               5

<210> 43
<211> 8
<212> PRT
<213> Escherichia coli

<400> 43

Thr His Gly Thr Ala Pro Lys Tyr
1               5

<210> 44
<211> 6
<212> PRT
<213> Escherichia coli

<400> 44
Met Met Leu Arg His Met
1               5

<210> 45
<211> 9
<212> PRT
<213> Artificial Sequence

<220>
<223> Description of Artificial Sequence:predicted
      ancestor sequence

<220>
<221> UNSURE
<222> (1)

<220>
<221> UNSURE
<222> (4)

<400> 45
Xaa Asp Leu Xaa Ala Asn Leu Arg Pro
1               5

<210> 46
<211> 10
<212> PRT
<213> Artificial Sequence

<220>
<223> Description of Artificial Sequence:predicted
      ancestor sequence

<220>
<221> UNSURE
<222> (4)

<220>
<221> UNSURE
<222> (6)

<220>
<221> UNSURE
<222> (9)

<400> 46
Ile Ala Arg Xaa Ala Xaa Phe Glu Xaa Ala
1               5                   10

<210> 47

<211> 8

<212> PRT

<213> Artificial Sequence

<220>

<223> Description of Artificial Sequence:predicted
      ancestor sequence

<400> 47

Val His Gly Ser Ala Pro Asp Ile
  1               5

<210> 48

<211> 6

<212> PRT

<213> Artificial Sequence

<220>

<223> Description of Artificial Sequence:predicted
      ancestor sequence

<220>

<221> UNSURE

<222> (4)..(6)

<400> 48

Met Met Leu Xaa Xaa Xaa
1               5

<210> 49
<211> 1014
<212> DNA
<213> Sulfolobus sp.

<220>
<221> CDS
<222> (1)..(1011)

<400> 49
atg ggc ttt act gtt gct tta ata caa gga gat gga att gga cca gaa     48
Met Gly Phe Thr Val Ala Leu Ile Gln Gly Asp Gly Ile Gly Pro Glu
1               5                   10                  15

ata gta tct aaa tct aag aga ata tta gcc aaa ata aat gag ctt tat     96
Ile Val Ser Lys Ser Lys Arg Ile Leu Ala Lys Ile Asn Glu Leu Tyr
            20                  25                  30

tct ttg cct atc gaa tat att gaa gta gaa gct ggt gat cgt gca ttg     144
Ser Leu Pro Ile Glu Tyr Ile Glu Val Glu Ala Gly Asp Arg Ala Leu
        35                  40                  45

gca aga tat ggt gaa gca ttg cca aaa gat agc tta aaa atc att gat     192
Ala Arg Tyr Gly Glu Ala Leu Pro Lys Asp Ser Leu Lys Ile Ile Asp
    50                  55                  60

aag gcc gat ata att ttg aaa ggt cca gta gga gaa tcc gct gca gac 240
Lys Ala Asp Ile Ile Leu Lys Gly Pro Val Gly Glu Ser Ala Ala Asp
65                  70                  75                  80

gtt gtt gtc aag tta aga caa att tat gat atg tat gcc aat att aga 288
Val Val Val Lys Leu Arg Gln Ile Tyr Asp Met Tyr Ala Asn Ile Arg
                85                  90                  95

cca gca aag tct atc ccg gga ata gat act aaa tat ggt aat gtt gat 336
Pro Ala Lys Ser Ile Pro Gly Ile Asp Thr Lys Tyr Gly Asn Val Asp
            100                 105                 110

ata ctt ata gtg aga gaa aat act gag gat tta tac aaa ggt ttt gaa 384
Ile Leu Ile Val Arg Glu Asn Thr Glu Asp Leu Tyr Lys Gly Phe Glu
        115                 120                 125

cat att gtt tct gat gga gta gcc gtt ggc atg aaa atc ata act aga 432
His Ile Val Ser Asp Gly Val Ala Val Gly Met Lys Ile Ile Thr Arg
    130                 135                 140

ttt gct tct gag aga ata gca aaa gta ggg cta aac ttt gca tta aga 480
Phe Ala Ser Glu Arg Ile Ala Lys Val Gly Leu Asn Phe Ala Leu Arg
145                 150                 155                 160

agg aga aag aaa gta act tgt gtt cat aag gct aac gta atg aga att 528
Arg Arg Lys Lys Val Thr Cys Val His Lys Ala Asn Val Met Arg Ile
                165                 170                 175

act gat ggt tta ttc gct gaa gca tgc aga tct gta tta aaa gga aaa 576
Thr Asp Gly Leu Phe Ala Glu Ala Cys Arg Ser Val Leu Lys Gly Lys

            180                 185                 190

gta gaa tat tca gaa atg tat gta gac gca gca gcg gct aat tta gta 624
Val Glu Tyr Ser Glu Met Tyr Val Asp Ala Ala Ala Ala Asn Leu Val
        195                 200                 205

aga aat cct caa atg ttt gat gta att gta act gag aac gta tat gga 672
Arg Asn Pro Gln Met Phe Asp Val Ile Val Thr Glu Asn Val Tyr Gly
    210                 215                 220

gac att tta agt gac gaa gct agt caa att gcg ggt agt tta ggt ata 720
Asp Ile Leu Ser Asp Glu Ala Ser Gln Ile Ala Gly Ser Leu Gly Ile
225                 230                 235                 240

gca ccc tct gcg aat ata gga gat aaa aaa gct tta ttt gaa cca gta 768
Ala Pro Ser Ala Asn Ile Gly Asp Lys Lys Ala Leu Phe Glu Pro Val
                245                 250                 255

cac ggt gca gcg ttt gac att gct gga aag aat ata ggt aat ccc act 816
His Gly Ala Ala Phe Asp Ile Ala Gly Lys Asn Ile Gly Asn Pro Thr
            260                 265                 270

gca ttt tta ctt tct gta agt atg atg tat gaa aga atg tat gag cta 864
Ala Phe Leu Leu Ser Val Ser Met Met Tyr Glu Arg Met Tyr Glu Leu
        275                 280                 285

tct aat gac gat aga tat ata aaa gct tca aga gct tta gaa aac gct 912
Ser Asn Asp Asp Arg Tyr Ile Lys Ala Ser Arg Ala Leu Glu Asn Ala
    290                 295                 300

ata tac tta gtc tac aaa gag aga aaa gcg tta acc cca gat gta ggt    960
Ile Tyr Leu Val Tyr Lys Glu Arg Lys Ala Leu Thr Pro Asp Val Gly
305                 310                 315                 320

ggt aat gcg aca act gat gac tta ata aat gaa att tat aat aag cta    1008
Gly Asn Ala Thr Thr Asp Asp Leu Ile Asn Glu Ile Tyr Asn Lys Leu
                325                 330                 335

ggc taa                                                            1014
Gly

<210> 50
<211> 337
<212> PRT
<213> Sulfolobus sp.

<400> 50
Met Gly Phe Thr Val Ala Leu Ile Gln Gly Asp Gly Ile Gly Pro Glu
  1               5                  10                  15

Ile Val Ser Lys Ser Lys Arg Ile Leu Ala Lys Ile Asn Glu Leu Tyr
             20                  25                  30

Ser Leu Pro Ile Glu Tyr Ile Glu Val Glu Ala Gly Asp Arg Ala Leu
         35                  40                  45

Ala Arg Tyr Gly Glu Ala Leu Pro Lys Asp Ser Leu Lys Ile Ile Asp
     50                  55                  60

Lys Ala Asp Ile Ile Leu Lys Gly Pro Val Gly Glu Ser Ala Ala Asp
65                  70                  75                  80

Val Val Val Lys Leu Arg Gln Ile Tyr Asp Met Tyr Ala Asn Ile Arg
                85                  90                  95

Pro Ala Lys Ser Ile Pro Gly Ile Asp Thr Lys Tyr Gly Asn Val Asp
            100                 105                 110

Ile Leu Ile Val Arg Glu Asn Thr Glu Asp Leu Tyr Lys Gly Phe Glu
        115                 120                 125

His Ile Val Ser Asp Gly Val Ala Val Gly Met Lys Ile Ile Thr Arg
    130                 135                 140

Phe Ala Ser Glu Arg Ile Ala Lys Val Gly Leu Asn Phe Ala Leu Arg
145                 150                 155                 160

Arg Arg Lys Lys Val Thr Cys Val His Lys Ala Asn Val Met Arg Ile
                165                 170                 175

Thr Asp Gly Leu Phe Ala Glu Ala Cys Arg Ser Val Leu Lys Gly Lys
            180                 185                 190

Val Glu Tyr Ser Glu Met Tyr Val Asp Ala Ala Ala Ala Asn Leu Val
        195                 200                 205

Arg Asn Pro Gln Met Phe Asp Val Ile Val Thr Glu Asn Val Tyr Gly
    210                 215                 220

Asp Ile Leu Ser Asp Glu Ala Ser Gln Ile Ala Gly Ser Leu Gly Ile
225                 230                 235                 240

Ala Pro Ser Ala Asn Ile Gly Asp Lys Lys Ala Leu Phe Glu Pro Val
                245                 250                 255

His Gly Ala Ala Phe Asp Ile Ala Gly Lys Asn Ile Gly Asn Pro Thr
            260                 265                 270

Ala Phe Leu Leu Ser Val Ser Met Met Tyr Glu Arg Met Tyr Glu Leu
        275                 280                 285

Ser Asn Asp Asp Arg Tyr Ile Lys Ala Ser Arg Ala Leu Glu Asn Ala
    290                 295                 300

Ile Tyr Leu Val Tyr Lys Glu Arg Lys Ala Leu Thr Pro Asp Val Gly
305                 310                 315                 320

Gly Asn Ala Thr Thr Asp Asp Leu Ile Asn Glu Ile Tyr Asn Lys Leu
                325                 330                 335

Gly

<210> 51
<211> 40
<212> DNA
<213> Artificial Sequence

<220>
<223> Description of Artificial Sequence:primer for site
      directed mutagenesis

<400> 51
tttgctggtc ttaagttggc ataaagatca taaatttgtc                    40

<210> 52
<211> 34
<212> DNA
<213> Artificial Sequence

<220>
<223> Description of Artificial Sequence:primer for site
      directed mutagenesis

<400> 52
agtttagccc tacgctcgcg attctctcag aagc                          34

<210> 53
<211> 31
<212> DNA
<213> Artificial Sequence

<220>
<223> Description of Artificial Sequence:primer for site
      directed mutagenesis

<400> 53
aatgcaaagt ttagcgctac ttttgctatt c                                    31

<210> 54
<211> 33
<212> DNA
<213> Artificial Sequence

<220>
<223> Description of Artificial Sequence:primer for site
      directed mutagenesis

<400> 54
tgcaaagttt agcgctactc ttgctattct ctc                                  33

<210> 55
<211> 32
<212> DNA
<213> Artificial Sequence

<220>
<223> Description of Artificial Sequence:primer for site
      directed mutagenesis

<400> 55
tccagcaatg tccggagcac taccgtgtac tg                                   32

<210> 56
<211> 29
<212> DNA
<213> Artificial Sequence

<220>
<223> Description of Artificial Sequence:primer for site
      directed mutagenesis

<400> 56
tcatacattc tctcgagcat catacttac                                    29

<210> 57
<211> 13
<212> PRT
<213> Neurospora crassa

<400> 57
Asp Pro Ile Thr Asp Glu Ala Leu Asn Ala Ala Lys Ala
 1               5                  10

<210> 58
<211> 13
<212> PRT
<213> Neurospora crassa

<400> 58

Val Trp Ser Leu Asp Lys Ala Asn Val Leu Ala Ser Ser
1               5                   10

<210> 59
<211> 7
<212> PRT
<213> Neurospora crassa

<400> 59
Lys Thr Lys Asp Leu Gly Gly
1               5

<210> 60
<211> 13
<212> PRT
<213> Saccharomyces cerevisiae

<400> 60
Val Pro Leu Pro Asp Glu Ala Leu Glu Ala Ser Lys Lys
1               5                   10

<210> 61
<211> 13
<212> PRT
<213> Saccharomyces cerevisiae

<400> 61

Ile Trp Ser Leu Asp Lys Ala Asn Val Leu Ala Ser Ser
1               5                   10

<210> 62
<211> 7
<212> PRT
<213> Saccharomyces cerevisiae

<400> 62
Arg Thr Gly Asp Leu Gly Gly
1               5

<210> 63
<211> 13
<212> PRT
<213> Agrobacterium tumefaciens

<400> 63
Val Ala Ile Ser Asp Ala Asp Asn Glu Lys Ala Leu Ala
1               5                   10

<210> 64
<211> 13
<212> PRT
<213> Agrobacterium tumefaciens

<400> 64

Val Cys Ser Met Glu Lys Arg Asn Val Met Lys Ser Gly
1               5                   10

<210> 65
<211> 7
<212> PRT
<213> Agrobacterium tumefaciens

<400> 65
Arg Thr Ala Asp Ile Met Ala
1               5

<210> 66
<211> 13
<212> PRT
<213> Bacillus subtilis

<400> 66
Asn Pro Leu Pro Glu Glu Thr Val Ala Ala Cys Lys Asn
1               5                   10

<210> 67
<211> 13
<212> PRT
<213> Bacillus subtilis

<400> 67

Val Thr Ser Val Asp Lys Ala Asn Val Leu Glu Ser Ser
1               5                   10

<210> 68
<211> 6
<212> PRT
<213> Bacillus subtilis

<400> 68
Arg Thr Arg Asp Leu Ala
1               5

<210> 69
<211> 13
<212> PRT
<213> Escherichia coli

<400> 69
Gln Pro Leu Pro Pro Ala Thr Val Glu Gly Cys Glu Gln
1               5                   10

<210> 70
<211> 13
<212> PRT
<213> Escherichia coli

<400> 70

Val Thr Ser Ile Asp Lys Ala Asn Val Leu Gln Ser Ser
1               5                   10

<210> 71
<211> 7
<212> PRT
<213> Escherichia coli

<400> 71
Arg Thr Gly Asp Leu Ala Arg
1               5

<210> 72
<211> 13
<212> PRT
<213> Thermus thermophilus

<400> 72
Glu Pro Phe Pro Glu Pro Thr Arg Lys Gly Val Glu Glu
1               5                   10

<210> 73
<211> 13
<212> PRT
<213> Thermus thermophilus

<400> 73

Val Val Ser Val Asp Lys Ala Asn Val Leu Glu Val Gly
1               5                   10

<210> 74
<211> 9
<212> PRT
<213> Thermus thermophilus

<400> 74
Glu Thr Pro Pro Pro Asp Leu Gly Gly
1               5

<210> 75
<211> 13
<212> PRT
<213> Sulfolobus sp.

<400> 75
Glu Ala Leu Pro Lys Asp Ser Leu Lys Ile Ile Asp Lys
1               5                   10

<210> 76
<211> 13
<212> PRT
<213> Sulfolobus sp.

<400> 76

Val Thr Cys Val His Lys Ala Asn Val Asn Arg Ile Thr
1               5                   10

<210> 77
<211> 9
<212> PRT
<213> Sulfolobus sp.

<400> 77
Lys Ala Leu Thr Pro Asp Val Gly Gly
1               5

<210> 78
<211> 13
<212> PRT
<213> Saccharomyces cerevisiae

<400> 78
Thr Thr Ile Pro Asp Pro Ala Val Gln Ser Ile Lys Thr
1               5                   10

<210> 79
<211> 13
<212> PRT
<213> Saccharomyces cerevisiae

<400> 79

Val Ser Ala Ile His Lys Ala Asn Ile Asn Gln Lys Thr
1               5                   10

<210> 80
<211> 9
<212> PRT
<213> Saccharomyces cerevisiae

<400> 80
Glu Asn Arg Thr Gly Asp Leu Ala Gly
1               5

<210> 81
<211> 13
<212> PRT
<213> Bos taurus

<400> 81
Trp Met Ile Pro Pro Glu Ala Lys Glu Ser Asn Asp Lys
1               5                   10

<210> 82
<211> 13
<212> PRT
<213> Bos taurus

<400> 82

Val Thr Ala Val His Lys Ala Asn Ile Asn Arg Met Ser
1               5                   10

<210> 83
<211> 9
<212> PRT
<213> Bos taurus

<400> 83
Asn Met His Thr Pro Asp Ile Gly Gly
1               5

<210> 84
<211> 13
<212> PRT
<213> Bacillus subtilis

<400> 84
Glu Trp Leu Pro Ala Glu Thr Leu Asp Val Ala Arg Glu
1               5                   10

<210> 85
<211> 13
<212> PRT
<213> Bacillus subtilis

<400> 85

Val Thr Leu Val His Lys Gly Asn Ile Asn Lys Phe Thr
1               5                   10

<210> 86
<211> 9
<212> PRT
<213> Bacillus subtilis

<400> 86
Arg Val Leu Thr Gly Asp Val Val Gly
1               5

<210> 87
<211> 13
<212> PRT
<213> Escherichia coli

<400> 87
Val Trp Leu Pro Ala Glu Thr Leu Asp Leu Ile Arg Glu
1               5                   10

<210> 88
<211> 13
<212> PRT
<213> Escherichia coli

<400> 88

Val Thr Leu Val His Lys Gly Asn Ile Asn Lys Phe Thr
1               5                   10

<210> 89
<211> 8
<212> PRT
<213> Escherichia coli

<400> 89
Val Val Thr Tyr Asp Phe Ala Arg
1               5

<210> 90
<211> 19
<212> DNA
<213> Artificial Sequence

<220>
<223> Description of Artificial Sequence:oligonucleotide
      for site directed mutagenesis

<400> 90
ctagttattg ctcagcggt                                                19

<210> 91
<211> 20
<212> DNA

<213> Artificial Sequence

<220>
<223> Description of Artificial Sequence:oligonucleotide
      for site directed mutagenesis

<400> 91
taatacgact cactataggg                                                    20

<210> 92
<211> 20
<212> DNA
<213> Artificial Sequence

<220>
<223> Description of Artificial Sequence:oligonucleotide
      for site directed mutagenesis

<400> 92
gggctcgggc aagggctcgc                                                    20

<210> 93
<211> 21
<212> DNA
<213> Artificial Sequence

<220>
<223> Description of Artificial Sequence:oligo

nucleotide for site directed mutagenesis

<400> 93
aggtccgggg tcggggtctc c                                                21

<210> 94
<211> 28
<212> DNA
<213> Artificial Sequence

<220>
<223> Description of Artificial Sequence:oligonucleotide
      for site directed mutagenesis

<400> 94
cttgtccacg ctcgtcacgt gcttcctg                                         28

<210> 95
<211> 32
<212> PRT
<213> Sulfolobus sp.

<400> 95
Val Ile Val Thr Glu Asn Val Tyr Gly Asp Ile Leu Ser Asp Glu Ala
 1               5                  10                  15

Ser Gln Ile Ala Gly Ser Leu Gly Ile Ala Pro Ser Ala Asn Ile Gly
            20                  25                  30

<210> 96

<211> 6

<212> PRT

<213> Sulfolobus sp.

<400> 96

Ala Leu Phe Glu Pro Val

1               5

<210> 97

<211> 32

<212> PRT

<213> Thermus thermophilus

<400> 97

Val Ile Val Thr Thr Asn Met Asn Gly Asp Ile Leu Ser Asp Leu Thr

1               5                   10                  15

Ser Gly Leu Ile Gly Gly Leu Gly Phe Ala Pro Ser Ala Asn Ile Gly

            20                  25                  30

<210> 98

<211> 6

<212> PRT

<213> Thermus thermophilus

<400> 98

Ala Ile Phe Glu Ala Val

1               5

<210> 99

<211> 32

<212> PRT

<213> Bos taurus

<400> 99

Val Leu Val Met Pro Asn Leu Tyr Gly Asp Ile Leu Ser Asp Leu Cys

1               5                   10                  15

Ala Gly Leu Ile Gly Gly Leu Gly Val Thr Pro Ser Gly Asn Ile Gly

            20                  25                  30

<210> 100

<211> 6

<212> PRT

<213> Bos taurus

<400> 100
Ala Ile Phe Glu Ala Val
1               5

<210> 101
<211> 33
<212> PRT
<213> Saccharomyces cerevisiae

<400> 101
Val Ser Val Cys Pro Asn Leu Tyr Gly Asp Ile Leu Ser Asp Leu Asn
1               5                   10                  15

Ser Gly Leu Ser Ala Gly Ser Leu Gly Leu Thr Pro Ser Ala Asn Ile
            20                  25                  30

Gly

<210> 102
<211> 6
<212> PRT
<213> Saccharomyces cerevisiae

<400> 102
Ser Ile Phe Glu Ala Val

1                 5

<210> 103

<211> 32

<212> PRT

<213> Caldococcus noboribetus

<400> 103

Val Ile Val Thr Pro Asn Leu Asn Gly Asp Tyr Ile Ser Asp Glu Ala

1                 5                   10                  15

Asn Ala Leu Val Gly Gly Ile Gly Met Ala Ala Gly Leu Asp Met Gly

                20                  25                  30

<210> 104

<211> 6

<212> PRT

<213> Caldococcus noboribetus

<400> 104

Ala Val Ala Glu Pro Val

1                 5

## Description

### Background of the Invention:

The present invention relates to a method for improving thermostability of a protein. The present invention also relates to a protein having an improved thermostability and a nucleic acid encoding the protein having improved thermostability.

A protein active at a high temperature, particularly a thermostable enzyme, is more advantageous than another protein which is inactivated at a high temperature, for example, in that it can be used without being cooled. Such a protein is mostly produced by a bacterium called thermophilic bacterium, which can grow at a high temperature. Accordingly, in designing a thermostable protein, amino acid sequence of a corresponding protein of such a group of thermophilic bacteria is analyzed and the characteristic feature of the amino acid sequence common to them is taken into account. Alternatively, the three-dimensional structure of a protein produced by the thermophilic bacterium is analyzed, the structure for imparting the thermostability is estimated from thus obtained information, and the structure of the heat-unstable protein is modified according to the estimated structure. As an example of proteins of thermophilic bacteria, 3-isopropylmalate dehydrogenase (IPMDH) encoded by *leu*B is known. The three-dimensional structure of IPMDH of *Thermus thermophilus* HB8 has been elucidated (K. Imada et al., J. Mol. Biol. 222, 725-738, 1991). Further, isocitrate dehydrogenase (ICDH) is known as a protein having a similar catalytic mechanism, amino acid sequence and three-dimensional structure as those of IPMDH, namely, a protein belonging to the same family as IPMDH.

### Summary of the Invention:

The object of the present invention is to provide a method for improving thermostability of protein, a protein having an improved thermostability and a nucleic acid encoding the protein, and host cells capable of producing a protein having improved thermostability.

In particular, the object of the present invention is to provide a method for improving thermostability of a protein, taking advantage of only the information of the primary structure of the protein.

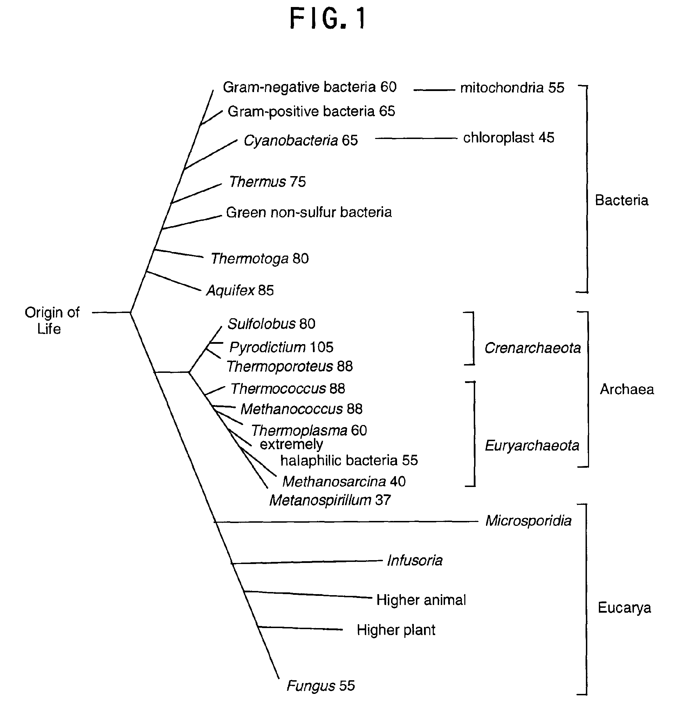

On the basis of the fact that many organisms which properly grow at a temperature of 80°C or above are located at the root of a phylogenetic tree by 16S rRNA (Fig. 1) shown by Woese et al., the inventors had an idea that the ancestors common to eubacteria, eukaryotes and archaebacteria might be ultra-thermophilic bacteria. On the basis of this supposition, the inventors have gotten an idea that although protein of many kinds of existing thermophilic bacteria are not always the protein of a true ancestral protein having an amino acid sequence of the ancestral or an amino acid sequence close to the ancestral sequence might have a further improved thermostability. The inventors have completed the present invention on the basis of an idea that for designing and producing a thermostable protein, it is more important that the amino acid sequence of ancestral protein is estimated and mimicked than that only the sequence and the higher-order structure of protein of a thermophilic bacterium are analyzed and mimicked.

Namely, the present invention provides a method for improving thermostability of proteins, which comprises the steps of
(i) comparing amino acid sequences of proteins derived from two or more species which evolutionarily correspond to each other in a phylogenetic tree;
(ii) estimating an amino acid sequence of an ancestral protein corresponding to the amino acid sequences compared in step (i); and,
(iii) and comparing the amino acid residues in the amino acid sequence in one of the proteins compared in step (i) with amino acid residues at a corresponding position in the ancestral protein estimated in step (ii), and replacing one or more of the amino acid residues different from those of the ancestral protein with the same amino acid residues as those of the ancestral protein.
   The present invention may further comprise the setps of
(iv) testing the proteins obtained in step (iii) for thermostability; and
(v) selecting a protein having improved thermostability.

The present invention particularly includes the comparison of species evolutionarily close to thermophilic bacteria or archaebacteria in the phylogenetic tree with each other on the amino acid sequence of corresponding proteins.

The present invention also provides an enzyme improved in heat resistance by the above-described method, a nucleic acid encoding the enzyme and host cells containing such a nucleic acid.

### Brief Description of the Drawings:

Fig. 1 shows a phylogenetic tree based on the comparison of 16S rRNA.
Fig. 2 shows the multiple alignment of amino acid sequences of IPMDH and ICDH from various biological species.
Fig. 3 shows a phylogenetic tree constructed by the simultaneous comparison of IPMDH and ICDH.
Fig. 4 shows the evolution of residue 152 of *Sulfolobus* sp. 7 strain.
Fig. 5 is a pE7-SB21 restriction enzyme map. pE7-SB21 was produced by inserting *leu*B gene into *Nde*I*-Eco*EI region of expression vector pET21c. Symbols in the figure represent the following restriction enzyme cleavage sites: N: *Nde* I, Sm: *Sma* I, E: *Eco*R I, E₄₇: *Eco*47 III, B: *Bgl* II, Xb: *Xba I,* H: *Hind* III, Xh: *Xho* I, and M: *Mro* I.
Fig. 6 shows the nucleotide sequence and amino acid sequence of *Sulfolobus* sp. *leu*B gene.
Fig. 7 shows the nucleotide sequence and amino acid sequence of *Sulfolobus* sp. *leu*B gene (continuation of Fig. 6).
Fig. 8 shows a rough variation introduction in abcd region. Symbols in the figure represent the following restriction enzyme cleavage sites: N: *Nde* I, Sm: *Sma* I, E: *Eco*R I, E₄₇: *Eco*47 III, *B: Bgl* II, Xb: *Xba* I, H: *Hind* III, Xh: *Xho I,* M: *Mro I,* Na: *Nae* I and Sa: *Sal* I.
Fig. 9 shows the multiple alignment of amino acid sequences of IPMDH and ICDH. The sequences with (ICDH) represent ICDH sequence and the sequences without the indication represent the IPMDH sequence. N. Cra: *Neurospora crassa*, *S.* Cer: *Saccharomyces cerevisiae,* A. tum: *Agrobacterium tumefacience,* B. sub: *Bacillus subtilis*, E. Col: *Escherichia coli,* T. The: *Thermus thermophilus*, Sub sp.#7: *Sulfolobus* stain #7, Cs. Cer: *Saccharomyces cerevisiae* (ICDH), CB. Tau: *Bos taurus*(ICDH), CB. Sub: *Bacillus subtilis*(ICDH), CE. Col: *Escherichia coli* (ICDH).
Fig. 10 shows the evolution of residue 53 of *Thermus thermophilus.*
Fig. 11 shows the scheme of mutagenesis using the plasmid containing cloned *Thermus thermophilus* IPMDH as a template.
Fig. 12 shows the residual activity of wild type *Thermus thermophilus* IPMDH and ancestral variants.

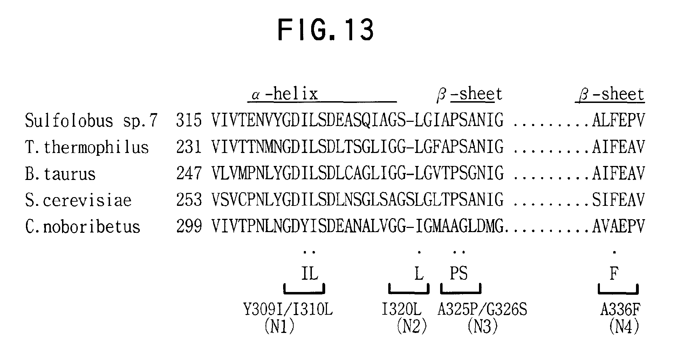

Fig. 13 shows the multiple alignment of IPMDH and ICDH.

### Description of the Preferred Embodiments:

Molecular phylogenetic tree (hereinafter referred to as "phylogenetic tree") based on the molecular level information of species or an algorithm for the preparation of the phylogenetic tree is utilized in the present invention. Some algorithms for preparing phylogenetic trees, such as the algorithm based on the maximum parsimony principle, are known. Computer programs for implementing the algorithms are utilizable or available. For example, various phylogenetic tree estimation programs such as CLUSTALW, PUZZLE, MOLPHY and PHYLIP are utilizable. Although phylogenetic trees can be produced by such programs, it is easier to utilize an already published phylogenetic tree (Fig. 1). For example, a phylogenetic tree based on 16S rRNA data proposed by Woese et al. is also usable. In such a phylogenetic tree, species which are close to each other in the molecular evolution appear in positions close to each other. Species positioned closely to the root of the phylogenetic tree are considered to be close to the ancestors.

For attaining the object of the present invention, it is preferred to use a part relatively close to the root of a phylogenetic tree, it is more preferred to use a part older than birds or even-toed ungulates, and it is particularly preferred to use a part of the phylogenetic tree which contains thermophilic bacteria or archaebacteria for the following reasons: The thermophilic bacteria and archaebacteria are positioned close to the root, namely, evolutionarily close to the ancestors in the phylogenetic tree. Further, proteins produced by them are expected to be relatively close to ancestral super-thermostable protein. It is also preferred to contain another protein belonging to the same family because ancestral amino acid residues (or sequence) at the root of the phylogenetic tree can be estimated, by a method which will be described below, by comparing the protein with a protein of archaebacteria or with another protein of the same family.

The term "thermophilic bacteria" is a generic name for bacteria capable of growing at a high temperature of usually above about 55°C. These bacteria are also called thermostable bacteria for the purpose of the present invention. In the present invention, the term "thermophilic bacteria" indicates both highly thermophilic bacteria capable of growing at a temperature of higher than above 75°C and also moderately thermophilic bacteria capable of growing at about 55 to 74°C. They also include facultative thermophilic bacteria capable of growing at ambient temperature and obligate thermophilic bacteria capable of growing only at a temperature of above about 40°C. The term "non-thermophilic bacteria" indicates microorganisms other than the thermophilic bacteria. The term "archaebacteria" indicates those classified according to the above-described Woese's classification. They indicate bacteria of prokaryote group including methane-forming bacteria, hyperhalophilic bacteria and sulphate reducing archaebacteria. The archaebacteria are clearly differentiated from eubacteria in that the lipid of the cell membrane of the former is an ether lipid. The expression "proteins belonging to the same family" herein indicates proteins which are similar to each other in at least one of the function, amino acid sequence, domain structure and steric structure. They include a group of proteins, at least amino acid sequences of which are partially homologous and the multiple alignment of which is possible. In particular, they include a group of proteins, at least amino acid sequences of which are homologous and can be multiple aligned. It is eagerly expected that two or more proteins belonging the same family are derived from the same ancestral protein.

Then information of amino acid sequences of proteins corresponding to each other, which are to be improved in thermostability, is obtained or determined from various species. Although proteins to which the present invention can be applied are not particularly limited, they are preferably proteins present in various species. Particularly enzymes having a high value of industrial utilization is preferable. Preferred examples of them are proteins produced by thermophilic bacteria, particularly thermostable enzymes. Example of them is IPMDH and ICDH of *Sulfolobus* sp. stain 7. The gene encoding IPMDH of this strain was cloned by Suzuki et al. [T. Suzuki et al., J. Bacteriol. 179 (4), 1174-1179, 1997].

Amino acid sequences of protein to be improved in thermostability can be also obtained from an already known data base. When an amino acid sequence is to be newly determined, any method for determining amino acid sequence known in the art can be employed. It is also possible to estimate the amino acid sequence by obtaining a nucleic acid encoding the protein according to the information of partial amino acid sequence, determining the nucleic acid sequence by a well-known sequencing techniques and estimating the amino acid sequence from the nucleic acid sequence.

After the multiple alignment of the obtained amino acid sequences from the species, the amino acid sequences obtained from the respective species are compared with each other. Some methods for the multiple alignment are known. One of the methods is based on the maximun parsimony principle for minimizing the change due to the insertion, deletion, replacement, etc. Computer programs for implementing this principle have been developed, which can be used or available. For example, TreeAlign is known among them. From DDBJ, "malign" which is the 1990 version of the program can be used. Because species which are evolutionarily close to each other in the phylogenetic tree are selected in the present invention, phylogenetic information has already been utilized in the multiple alignment and, as a result, the alignment is more suitable than that in a case of no phylogenetic information can be conducted. Information from at least three species is utilized for the multiple alignment. The larger the number of origin of the data to be used for the alignment, the more suitable the information. Furthermore, each of the species to be compared preferably contains one or more thermophilic bacteria or archaebacteria, based on the aforementioned reason. It is also preferred that it contains a family protein, namely another protein expected to be derived from the same ancestral protein.

After obtaining the results of the alignment, amino acid sequence of the ancestral protein can be estimated on the phylogenetic tree. For this purpose, the maximum parsimony method or maximal likelihood method is utilizable. The procedure of such a method is well known to those skilled in the art [see, for example, Young, Z., Kumar, S and Nei. M, Genetics 141, 1641-16510, 1995; Steward, C. -B. Active ancestral molecules, Nature 374, 12-13, 1995; and Molecuar Evolutinary Genetics, Columbia University Press, New York, USA, 1987]. For example, the maximal parsimony method which can be employed in the present invention is, in short, a method wherein an ancestral type having the minimal number of the mutation expected to occur after the estimation of the ancestral type is likely estimated to be the true ancestral type. The maximal likelihood method can be employed instead of the maximum parsimony method. Also, a program PROTPARS (included in PHYLIP) for directly estimating the ancestral type from the amino acid sequence according to the maximum parsimony method can be also employed. Because the phylogenetic tree and ancestral amino acid are principally estimated at the same time in those methods, it is not always necessary to prepare the phylogenetic tree when such a method is employed. However, the preparation of the phylogenetic tree is preferred particularly when the ancestral amino acid is to be estimated by manual calculation. The ancestral amino acid sequence can be determined by the following maximum parsimony method or maximal likelihood method according to a phylogenetic tree produced by the above-described method or another already known method, particularly based on an already published phylogenetic tree.

A process according to the maximum parsimony method will be described in detail with reference to IPMDH which will be shown also in Examples given below.

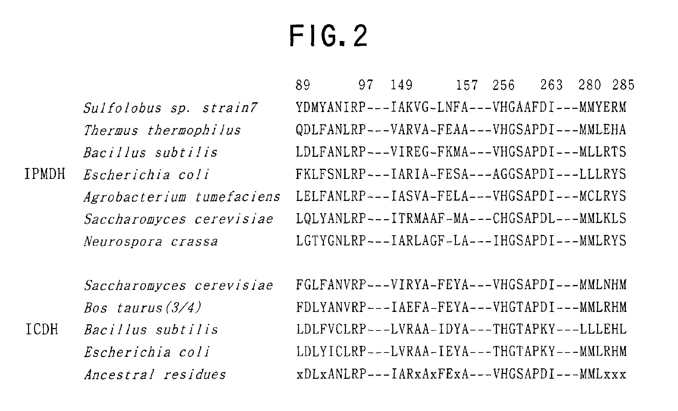

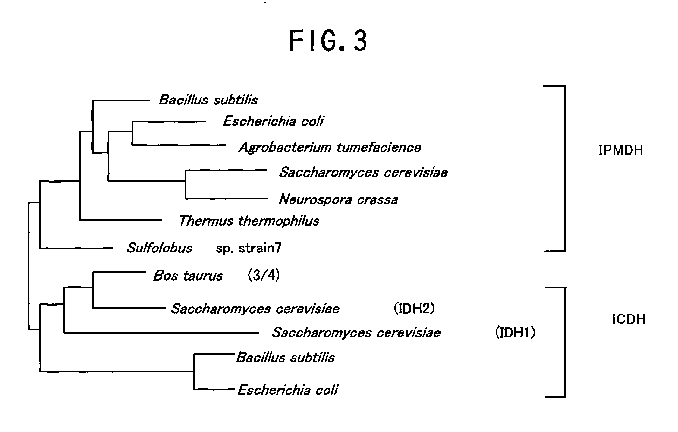

Amino acid sequences from some species of IPMDH and ICDH, which have already been cloned and of which sequences were determined, are multiply aligned (Fig. 2). Then a phylogenetic tree is prepared on the basis of the sequences by, for example, the maximum parsimony method or neighbor-joining method (Fig. 3). In this case, it is possible to directly estimate the ancestral amino acid sequence, without preparing the phylogenetic tree, by the maximum parsimony method as described above. However, a procedure wherein the phylogenetic tree is explicitly used will be described for easy understanding of the procedure. This procedure is also applicable to a case when an already prepared phylogenetic tree such as a published known phylogenetic tree is used.

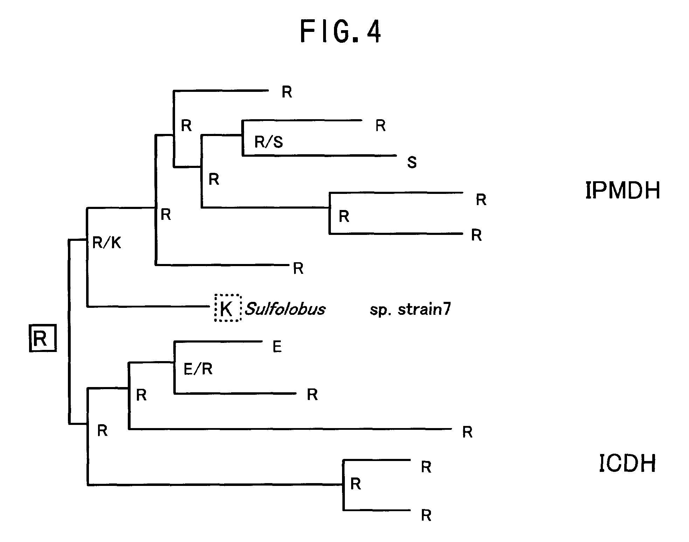

Ancestral amino acids in respective sites of the multiply aligned residues can be determined by means of a phylogenetic tree obtained by any method. For example, Fig. 4 shows amino acid residues from various organisms corresponding to residue 152 of *Sulfolobus* sp. strain 7 of IPMDH. Amino acids at this position in the organisms shown in Fig. 4 are R, S, K or E. When both residues in species close to each other in the phylogenetic tree are R, it can be estimated that in the ancestral species common to them (shown by the binding point connecting two species in the phylogenetic tree), the amino acid residue corresponding to residue 152 of *Sulfolobus* sp. strain 7 would be R for the following reasons: When R is the ancestral type, only one variation can elucidate the mechanism of the realization of the amino acid residue corresponding to residue 152 of *Sulfolobus* sp. strain 7 in the present species, while when S is the ancestral type, two or more times of variation must be taken into consideration.

When two species have residues different from each other, such as residues R and S, the ancestor common to both of them cannot be immediately determined. However, even in such a case, the common ancestor can be estimated to be R when another branch in one branch deeper position (i. e. junction on the left-hand side in the phylogenetic tree) is R. Thus, the amino acid sequence on the most left-hand side in the figure can be estimated to be the most ancestral amino acid sequence by evolutionarily tracing back (i. e. going back to the left in the figure). In Fig. 4, the ancestral amino acid residue corresponding to residue152 of *Sulfolobus* sp. strain 7 is estimated to be R.

By thus estimating the ancestral amino acid residue of each residue in the sequence in the multiple alignment, the ancestral amino acid sequence in a corresponding region can be estimated. When the species used for the estimation of the ancestral amino acid sequence is changed, the shape of the phylogenetic tree is changed and, therefore, a different ancestral amino residue is obtained in some cases. The position and variety thereof are variable also depending on the protein used for the comparison. Therefore, for attaining the object of the present invention, it is preferred to alter an amino acid residue selected at a position of a relatively slight change. Such an amino acid residue can be determined by changing the species used for the preparation of the phylogenetic tree or by using only a part of amino acid sequence information used for the preparation of the phylogenetic tree without changing the species, and estimating the degree of the change in shape of the tree due to the change of the amino acid sequence information used for preparing the phylogenetic tree and selecting a residue which only slightly influence on the shape of the tree.

As far as various species have regions corresponding to each other, the ancestral amino acid sequence in the regions can be estimated in proteins to be improved in the thermostability by the above-described procedure. Each amino acid residue in thus determined amino acid sequence may correspond to amino acid residues in many positions in a protein of a present species of organism particularly when the organism is a thermophilic bacterium or archaebacteria. Accordingly, in the present invention, only amino acid residues having a sequence different from that of the ancestral protein amino acid sequence are to be modified in such a case.

In the estimation of the amino acid sequence of protein of ancestral species according to the above-described procedure, the ancestral type can be determined by the above-described procedure irrespective of the fact that a thermophilic bacterium or non-thermophilic bacterium is contained in the species to be compared or the fact that only the thermophilic bacterium has an amino residue different from that of other species to be compared. When there are many species having proteins having amino acid sequences different from others and, therefore, the ancestral type cannot be estimated only from the information or the degree of accuracy is considered to be low, data for the alignment can be further added. When the ancestral amino acid residue can be thus determined, this amino acid residue can be employed as the ancestral one.

Generally, two or more positions and regions having such amino acid residues may present in the protein. These positions and regions might be either apart from one another or close to one another. All of these positions and amino acid residues are recorded for the modification which will be described below.

After the determination of the ancestral amino acid residue for the amino acid residue at each position, at least one of non-ancestral amino acid residues of the protein to be analyzed is replaced with the ancestral amino acid residue to modify the protein. In this case, the number and position of the amino acid residues to be replaced may vary depending on the protein to be modified, required thermostability and desired specific activity. Preferably, the position and number of the amino acid residues to be replaced are selected so that both sufficient thermostability and high specific activity can be attained. For obtaining both sufficient thermostability and high specific activity at the same time, further information of the position of the active center and amino acid sequence around the active center is useful.

Although the protein to be modified can be derived from any of the comparative species, it is preferred to select protein from species having the highest thermostability. It is particularly preferred to select a protein produced by the thermophilic bacterium as the protein to be modified for the following reasons: A protein from a species of organism having a high thermostability is generally expected to have a high thermostability. Further, by modifying a protein expected to already have certain thermostability to a more complete ancestral protein, a further improvement in the thermostability can be expected. The amino acid residues in a protein can be replaced by altering a nucleic acid encoding the protein. In short, the site-specific mutagenesis by Kunkel method can be conducted by obtaining a gene encoding the protein in which the amino acid residue is to be replaced and using a primer capable of replacing an amino acid residue in an intended site. Further, the site-specific mutagenesis can be carried out by a PCR method.

An intended gene can be obtained by a hybridization method or PCR after designing a suitable probe according to a known amino acid sequence information or a partial amino acid sequence information of the protein. DNA having an intended mutation can be efficiently replicated by previously preparing a template for the mutagenesis in ung⁻ host. It is convenient for the confirmation of the mutation when a primer for the mutagenesis is designed to have a restriction enzyme site.

The molecular biological techniques such as introduction of a gene into a host, cloning of genes and site-specific mutagenesis including ung⁻ hosts, are well known by those skilled in the art. For these techniques, for example, Sambrook et al., 1989, Molecular Cloning: A Laboratory Manual, Second Edition (1989) Cold Spring Harbor Laboratory Press, Cold Spring Harbor, New York, and F. M. Ausubel et alo. (eds), Current Protocols in Molecular Biology, John Wiley & Sons, Inc. (1994) can be referred to. Further, kits for carrying out these molecular biological techniques are commercially available. The mutation thus introduced can be confirmed by determining the nucleotide sequence. When a restriction enzyme site has been introduced in the primer for the variation introduction, the introduction of the mutation can be more easily confirmed on the basis of the fact that it can be digested by a corresponding restriction enzyme.

The modified gene thus obtained can be expressed with a suitable host-vector system. The hosts usable herein include both eucaryotic cells and procaryotic cells. Generally, microorganisms such as *Escherichia coli* are preferred. Recombinant DNA molecules prepared by introducing the modified gene into an expression vector having a regulatory sequence required for expressing the modified gene depending on the selected host can be prepared. Such an expression vector is well known in the art, and many host - vector systems are available on the market. Among those vectors, usually host - vector high expression systems are preferred. Inducible host - vector systems are particularly preferred. However, the selection of a suitable host - vector system will vary depending on the properties of protein because some proteins will harm the host upon the high expression. If necessary, the codon usage may be optimized depending on the selected host. The host containing such a recombinant DNA molecule may be cultured using a method well known in the art and then the produced protein may be recovered.

The protein can be recovered from the host cells or culture medium by an ordinary method selected depending on the host and properties of the produced protein. For example, when the protein is recovered from the microbial cells, the cells are broken by, for example, sonication, the residue is removed by centrifugation and the intended protein is obtained by a proper combination of ammonium sulfate precipitation, reversed phase chromatography, ion exchange chromatography, gel filtration, etc. When the protein is in the form of an inclusion body, it can be solubilized with 6 M guanidine hydrochloride or the like and reconstituted. When the protein is recovered from the culture medium, the microbial cells are removed by centrifugation and then the intended protein is recovered in the same manner as that described above. When the intended protein has a property of being associating with the cell membrane, a suitable surfactant can be used for the solubilization. The solubilization methods are well known in the art, and they are suitably selected depending on the properties of the protein.

The purity of the obtained protein can be confirmed by, for example, SDS-polyacrylamide gel electrophoresis. The concentration of the obtained protein can be determined by a method well-known in the art, for example using BCA Protein Assay Kit from PIERCE Co., wherein bovine serum albumin is used as the standard protein, as will be described in Examples given below. The thermostability of the protein can be determined by examining the activity thereof after the heat treatment. For example, the thermostability of IPMDH can be determined by the following method: An assay buffer (50 mM CHES/KOH, pH 9.5, 200 mM KCI, 1 mM NAD, 0.4 mM IPM, 5 mM MgCl₂) was introduced into a cell and then incubated at an appropriate temperature, for example 50°C-99°C for 5 minutes. A suitable amount of an enzyme solution having a suitably prepared concentration is added to the assay buffer and the obtained mixture is lightly stirred. The mixture is kept at 50°C-75°C and the increase in NADH is determined by the ultraviolet absorbance at 340 nm. The specific activity of IPMDH is shown in terms of units (U) per mg of protein. The activity for producing 1 micromole of NADH per minute 75°C can be represented to be 1 U (unit).

For ICDH the thermostability can be determined by the following method: An assay buffer (10 mM MgCl₂,0.4mM D,L-isocitrate, 0.8mM NADP, 100mM PIPES pH 7.0) was introduced into a cell and then incubated at a high temperature, for example 50°C-99°C for 5 minutes. A suitable amount of an enzyme solution having a suitably prepared concentration is added to the assay buffer and the obtained mixture is lightly stirred. The mixture is kept at 50°C-75°C and the increase in NADPH is determined by the ultraviolet absorbance at 340 nm. The activity for producing 1 micromole of NADPH per minute 70°C can be represented to be 1 U (unit).

Thus, ancestral variants may be optionally tested for thermostability by determining their activity at high temperature with suitable methods to select more thermostable proteins.

### Examples

Strains and culture media shown below were used.

### (1) Escherichia coli

CJ236: This strain was used for preparing uracil single strand DNA (UssDNA). This strain is defective in uracil glycosylase and dUTPase.

MC1061 and JM109: They were used as hosts in the gene operation.

MA153: This strain was used as the host for large scale expression of IPMDH. This strain is defective in *leuB.*

### (2) Media

LB agar medium: 1.0 % of bactotryptone, 0.5 % of bactoyeast extract, 1 % of NaCI, 1.5 % of agar and, if necessary, 100µg/ml of ampicillin.

M9 agar medium: 1xM9 salt, 1 mM of MgSO₄, 0.1 mM of CaCl₂, 0.001 % of thiamine, 0.2 % of glucose and 1.5 % of agar. This medium was used for the selection of *Escherichia coli* JM109.

2xYT medium: 1.6 % of bactotryptone, 1.0 % of bactoyeast extract and 0.5 % of NaCl. This medium was used for the liquid culture of *Escherichia coli*. If necessary, 100 µg/ml of ampicillin was added.

### (3) Determination of IPMDH activity:

490 µI of an assay buffer (50 mM of CHES/KOH, pH 9.5, 200 mM of KCI, 1 mM of NAD, 0.4 mM of IPM and 5 mM of MgCl₂) was fed into a cell and then preincubated at 50°C-75°C for 5 minutes. Then 10 µI of an enzyme solution having a predetermined concentration was added thereto, and the obtained mixture was lightly stirred. Then keeping the mixture at the same temperature as the preincubation temperature, an increase in NADH was determined according to the ultraviolet absorbance at 340 nm.

### (4) Determination of ICDH activity:

490 µl of an assay buffer (10 mM of MgCl₂, 0.4mM D.L-isocitrate, 0.8mM NADP, 100mM PIPES pH7.0) was fed into a cell and then preincubated at 50°C-75°C for 5 minutes. Then 10 µI of an enzyme solution having a predetermined concentration was added thereto, and the obtained mixture was lightly stirred. Then keeping the mixture at the same temperature as the preincubation temperature, an increase in NADPH was determined according to the ultraviolet absorbance at 340 nm.

### Example 1 Construction of ancestral IPMDH from Sulfolobus sp. strain 7

### (1) Preparation of uracil single-strand DNA (UssDNA)

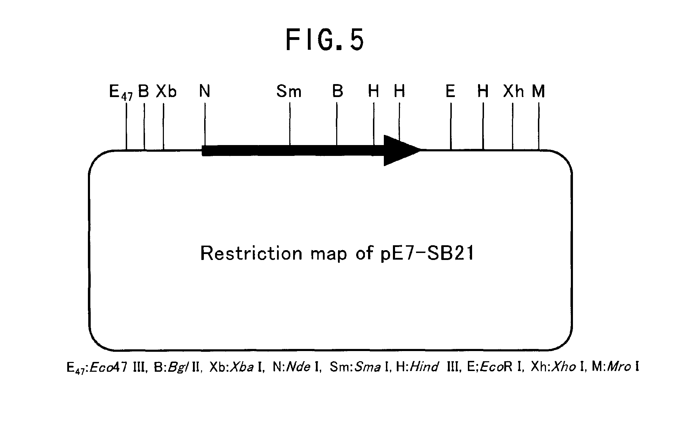

*leuB* expression plasmid pE7-SB21 (Fig. 5) was introduced into competent cells of *E*. *coli* CJ236. The obtained transformed CJ236 was cultured in 2xYT medium to obtain 30 ml of a liquid culture. CJ236 in the liquid culture was infected with helper phage M13KO7. After shaking the culture in 2xYT medium at 37°C for 5 hours, the obtained culture was centrifuged at 5,000 rpm at 4°C for 10 minutes. The supernatant was further centrifuged at 6,000 rpm at 4°C for 10 minutes to obtain a supernatant. A phage was precipitated from 10 ml of the supernatant by PEG/NaCl. 10.9µg of UssDNA was obtained from the phage by an ordinary method. The concentration was 363 µg/ml.

### (2) Estimation of amino acid sequence of ancestral IPMDH

Amino acid sequences of IPMDH and ICDH which had been cloned and the amino acid sequences of which had been made clear were subjected to the multiple alignment. The results are shown in Table 1. Then, the ancestral amino acid sequences in respective regions (regions a, b or b' and b", c and d) shown in Table 1 were estimated. The estimation was conducted by the above-described procedure. For example, residue 152 was estimated as will be described below.

At first, a phylogenetic tree containing these species was prepared by the neighbor-joining method (Fig. 3). Then b regions of *Saccharomyces cerevisiae* and *Neurospora crassa* in the phylogenetic tree were compared with each other. The amino acid residues corresponding to residue 152 of *Sulfolobus* sp. strain 7 were R in these two species. Accordingly, amino acid residues at the corresponding positions of the two ancestral species were estimated to be R. Then *Escherichia coli* and *Agrobacterium tumefaciens* were compared with each other to find that the amino acid residues corresponding to residue 152 of *Sulfolobus* sp. strain 7 were R and S, respectively. Therefore, amino acid residues at corresponding positions of the two ancestral species could not be estimated from only this fact. However, at the junction in the left branch, the amino acid residue was estimated to be R in another branch (i. e. branch which branches into *Saccharomyces cerevisiae* and *Nuerospora crassa)* as described above. Accordingly, the amino acid residue at this position in four common ancestral species, i. e. *Saccharomyces cerevisiae, Nuerospora crassa, Escherichia coli* and *Agrobacterium tumefaciens,* was estimated to be R. Further, because amino acid residue of *Bacillus subtilis* corresponding to residue 152 of *Sulfolobus* sp. strain 7 was R, it was estimated that amino acid residue in the corresponding position in the ancestral species of 5 organisms (the above-described 4 organisms and *Bacillus subtilis)* was estimated to be R. By thus tracing back to the left in the phylogenetic tree in Fig. 5, it was estimated that the amino acid residue corresponding to position 152 of *Sulfolobus* sp. strain 7 would be R.

By repeating the procedure, the ancestral amino acid sequence for the amino acid sequences in the domains shown in Table 1 was finally determined. Then thus determined ancestral amino acid sequence was compared with the amino acid sequence of *Sulfolobus* sp. strain 7 to determine the amino acid residue and position thereof of *Sulfolobus* sp. strain 7 different from the ancestral sequence. As a result, it was found that the amino acid residue and position thereof of each of M91, 195, K152, G154, A259, F261 and Y282 were different from those of the ancestral type. As for these symbols, for example, M91 represents M (methionine) residue at position 91. The same shall apply to other symbols.

In Table 1, these residues are underlined. The ancestral amino acid sequences determined by the above-described procedure and the positions and varieties of amino acid residues to be modified are also shown in Table 1. Residues shown by "x" in Table 1 are positions at which the ancestral type was not only one.

From these results, it was determined that in the ancestral enzyme, amino acid residue at position 91 was L, amino acid residue at position 95 was L, amino acid residue at position 152 was R, amino acid residue 154 at position was A, amino acid residue at position 259 was S, amino acid residue at position 261 was P and amino acid residue at position 282 was L.

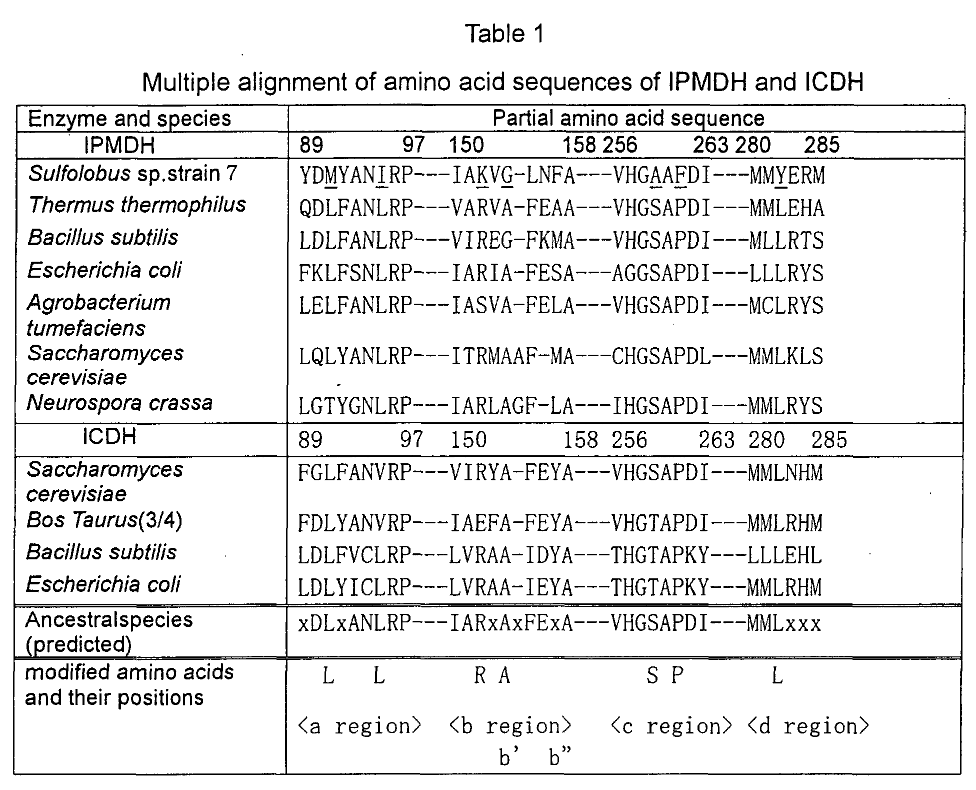

The partial amino acid sequences in the above Table are shown as sequence SEQ ID:1 to SEQ ID:48 in order in the sequence listing.

### (3) Design of primer for the mutagenesis

After the amino acid sequences of ancestral IPMDH and ICDH were determined, some ancestral variants were prepared by replacing amino acid residues in regions a, b, c and d and the combinations of them. The amino acid residue replacement in the ancestral variants was as follows: ancestral variation in a region (M91L and I95L), ancestral variation in b' region (K152R), ancestral variation in b" region (G154A), ancestral variation in b region (K152R and G154A), ancestral variation in c region (A259S and F261P), ancestral variation in d region (Y282L), and ancestral variation in a, b, c and d region (M91L, I95L, K152R, G154A, A259S, F2651P and Y282L). As for these symbols, for example, M91L represents the replacement of M (methionine) residue at position 91 with L (leucine) residue. The same shall apply to other symbols.

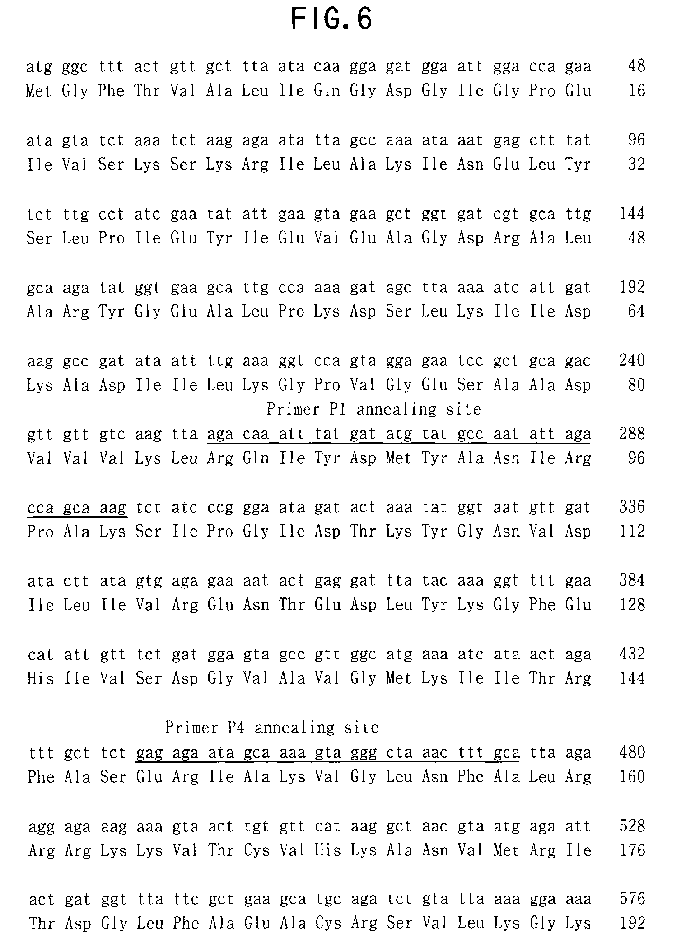

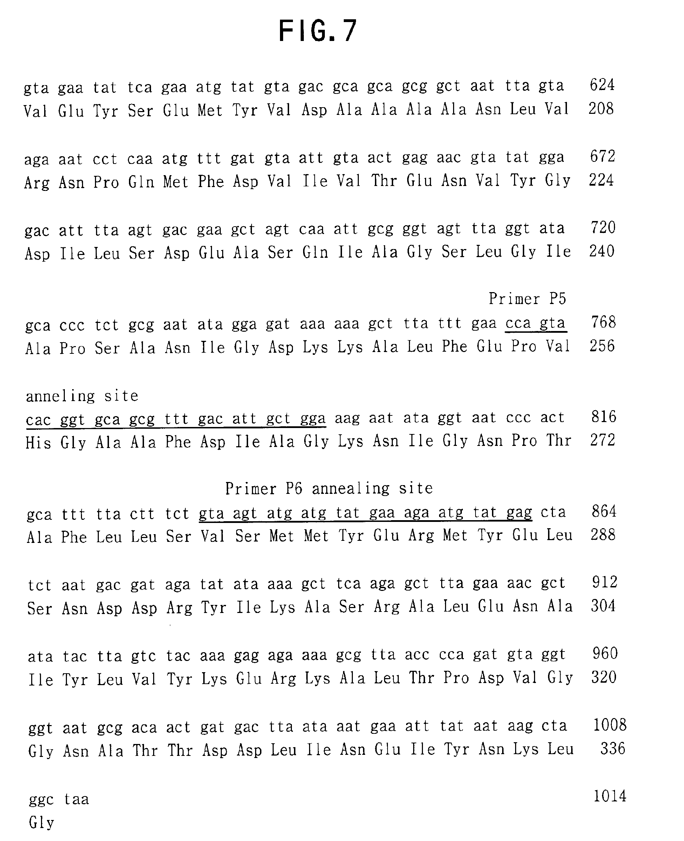

Primers shown below were designed for preparing these ancestral variants using a site-specific mutagenesis method. The respective primers were designed with reference to the nucleotide sequence (SEQ ID:49) and amino acid sequence (SEQ ID:50) of IPMDH of *Sulfolobus* sp. strain 7 (Figs. 6 and 7).
Primer P1 for introduction of ancestral mutation in a domain 
5'-TTTGCTGGTCTTAAGTTGGCATAAAGATCATAAATTTGTC-3'
(SEQ ID:51)
 (The underlined part is the site of recognition of restriction enzyme *Af*/II)
Primer P2 for introduction of ancestral mutation in b' domain 5'-AGTTTAGCCCTACGCTCGCGATTCTCTCAGAAGC-3' (SEQ ID:52) (The underlined part is the site of recognition of restriction enzyme *Nru*I)
Primer P3 for introduction of ancestral mutation in b" domain 5'-AATGCAAAGTTTAGCGCTACTTTTGCTATTC-3' (SEQ ID:53) (The underlined part is the site of recognition of *Eco*47 III)
Primer P4 for introduction of ancestral double mutation in b domain 5'-TGCAAAGTTTAGCGCTACTCTTGCTATTCTCTC-3' (SEQ ID:54) (The underlined part is the site of recognition of *Eco*47 III)
Primer P5 for introduction of ancestral mutation in c domain 5'-TCCAGCAATGTCCGGAGCACTACCGTGTACTG-3' (SEQ ID:55) (The underlined part is the site of recognition of *Mro* I)
Primer P6 for introduction of ancestral mutation in d domain 5'-TCATACATTCTCTCGAGCATCATACTTAC-3'    (SEQ ID:56) (The underlined part is the site of recognition of *Xho I)*

Because abcd ancestral mutation includes all the mutations introduced by the combination of the above-described primers, no primer was prepared.

### (4) Introducing the mutations by Kunkel method

Each of the primers having the sequence of SEQ ID:3 to SEQ ID:8 was dissolved in TE (10 mM Tris-HCI, 1 mM EDTA, pH 8.0) by an ordinary method to obtain 10 pmol/µl solution. 1 µl of the primer solution (the total: 10 µl) was phosphorylated with polynucleotide kinase by an conventional method. After the completion of the reaction, the enzyme was inactivated by the treatment at 70°C for 10 minutes. 3 µl of the reaction liquid was taken and mixed with 1.5 µl of UssDNA obtained in step (1) and was allowed to anneal. Thus the mixture contained all the primers of phosphatized sequence Nos. 3 to 8. The annealing step was conducted in the total amount of 20µl containing 10 x annealing buffer (200 mM Tris-HCI, 20 mM MgCl₂, 100 mM DTT, pH 8.0). The mixture was heated to 70°C and then left to stand at room temperature to cool it to about 30°C.

After annealing, 2 µl of 10 x synthetic buffer (50 mM Tris-HCI, 20 mM MgCl₂, 5 mM dNTPs, 10 mM ATP, 20 mM DTT, pH 7.9), 1µl of T4 DNA ligase and 1µl of T4 DNA polymerase were added to the annealed solution. The obtained mixture was kept in ice for 5 minutes and then at room temperature for 5 minutes, and then incubated at 37°C for 90 minutes. 4µl of the reaction mixture was taken and mixed with 100 µl of *Escherichia coli* MC 1061 competent cells. The obtained mixture was left to stand at 0°C for 20 minutes, at 42°C for 1 minute and 0°C for 2 minutes. 450µl of 2xYT medium was added thereto and they were left to stand at 37°C for 1 hour. 138.5µl of of the culture liquid was poured into 5 ml of 2xYT liquid medium containing 100µg /ml of ampicillin. After overnight culture, the plasmid DNA was recovered from the cells by alkali-SDS method.

*Escherichia coli* MC1061 was again transformed by DNA thus obtained. Transformed colonies were selected on LB agar medium containing 100µg/ml of ampicillin. The colonies were cultured and plasmid DNA was recovered therefrom to confirm whether the site of the restriction enzyme was found or not. When the mutation was introduced, DNA would be digested by the restriction enzyme in the primer corresponding to the mutation site.

As a result, several plasmids having ancestral variation introduced into the above-described regions a to d or a combination of them were obtained.

In the variants thus obtained, (M91L and I95L) ancestral variant, (K 152 R) ancestral variant, (G154A) ancestral variant, (K152R and G154A) ancestral variant, (A259S and F261P) ancestral variant and (Y282L) ancestral variant were named a variant, b' variant, b" variant, b variant, c variant and d variant, respectively, and also corresponding expression plasmids were named pE7-SB21a, pE7-SB21b', pE7-SB21b", pE7-SB21b, pE7-SB21c and pE7-SB21d, respectively.

Because ancestral variant in abcd region was not obtained, however, this variant was constructed from the ancectral a region variant and ancestral bcd region variant.

Ancestral bcd region variant plasmid pE7-SB21bcd DNA obtained as described above was digested with *Sma* I. On the other hand, a variant plasmid pE7-SB21a DNA was digested with *Xba* I and *Eco* RI, and DNA segment encoding the intended enzyme was subcloned into *Xba* I *- Eco* RI multicloning site of pUC118 to obtain plasmid pUC118-SB21a. pUC118-SB21a was digested with *Sma* I and ligated with the above-described bcd rgion ancestral variant plasmid DNA digested with *Sma* I to obtain pUC118 -SB21abcd. Then pUC118-SB21abcd and pE7-SB21 were digested with *Xba* I and *Eco* RI. They were mixed together to obtain expression plasmid pE7-SB21abcd for the ancestral variant in abcd region.

The fact that pE7-XB21a, pE7-SB21b', pE-7-SB21b", pE7-SB21b, pE7-SB21c, pE-SB21d and pE7-SB21abcd had the intended ancestral variants was confirmed by examining the presence or absence of a cleavage site of the corresponding restriction enzyme and determining the nucleotide sequence.

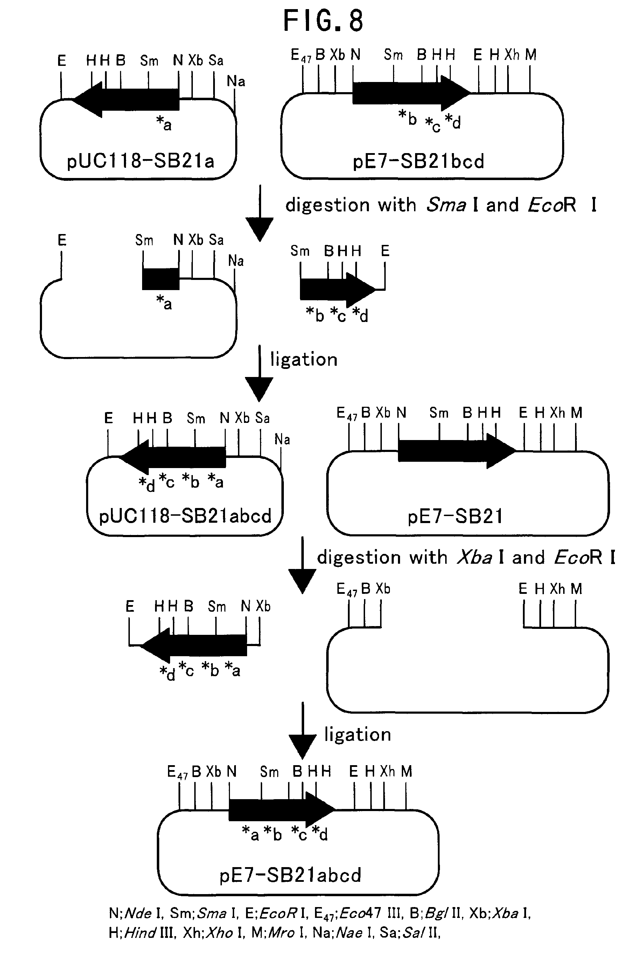

Fig. 8 shows a schematic diagram of the construction of the plasmids.

### Example 2 Purification of Sulfolobus sp. IPMDH and ancestral IPMDH

Colonies of *Escherichia coli* MA153 having plasmid of natural type or ancestral variant were taken in 100 ml of 2xYT medium containing 100µg/ml of ampicillin. After culturing overnight, they were each inoculated to 10 liters of 2 xYT medium containing 100µg/ml of ampicillin. After culturing by shaking at 37°C until OD₆₀₀ = 0.6, IPTG was added so as to obtain a final concentration of 0.4 mM. After culturing by shaking for additional 2 hours, the microbial cells were recovered by the centrifugation at 7,000 rpm at 4°C for 10 minutes. The obtained microbial cells were suspended in buffer I (20 mM KHPO₄, 0.5 mM EDTA, pH 7.0) and cleaned by the centrifugation at 7,000 rpm at 4°C for 20 minutes. When the next step was not immediately started, the cells were kept at -80°C. 19.6 g of the microbial cells were obtained.

2 parts of buffer I containing 1 mM DTT was added to 1 part of the microbial cells to obtain a suspension. The suspended cells were crushed by sonication, and the precipitate was removed by the centrifugation at 30,000 rpm at 4°C for 20 minutes. The supernatant was heat-treated at 75°C for 20 minutes and then centrifuged at 30,000 rpm at 4°C for 20 minutes. Modified protein thus precipitated was removed.

The supernatant was treated with anion exchange column DE-52 equilibrated with Buffer I, and the passed fraction was recovered. 3 M ammonium sulfate (AS) solution was added to the obtained fraction to obtain the final concentration of 1 M. After leaving the mixture to stand at 4°C for about 1 hour, the precipitates thus formed were removed by the centrifugation at 30,000 rpm at 4°C for 20 minutes. The supernatant was passed through butyl-Toyopearl 650 s column (a hydrophobic column) equilibrated with Buffer I containing 1 M of AS. Protein was eluted by the linear inclination of AS concentration of 1 M to OM. The activity of each of the obtained fractions was determined. The active fractions were collected and dialyzed against Buffer II (20 mM CHES/KOH, 0.5 mM EDTA, pH 9.3).

The protein solution obtained by the dialysis was treated with a Resource Q column (an anion exchange column) equilibrated with Buffer II and protein was eluted by the linear gradient of KCI concentration of 0 M to 0.1 M. Each fraction thus obtained was dialyzed against Buffer I and the purity was confirmed with SDS-PAGE. Fractions of a single band confirmed with SDS-PAGE were collected and concentrated to 1 mg/ml with Cetnriprep 30. The protein concentration was determined using BCA protein assay reagent kit of PIERCE Co. with BSA as the standard. The purification results are shown in Table 2.

**Table 2**

| 19.67g of microbial cells | Total activity (U) | Yield (%) | Protein (mg) | Specific activity (U/mg) | Relative Purity |
|---|---|---|---|---|---|
| Crude extract | - | - | 2278.3 | - | - |
| After heating | 34.74 | 100.0 | 230.5 | 0.15 | 1.00 |
| DE-52 | 33.93 | 97.7 | 80.67 | 0.42 | 2.80 |
| Butyl-Toyopearl | 33.72 | 97.1 | 7.12 | 5.02 | 33.47 |
| Resource Q | 15.05 | 43.3 | 1.60 | 11.00 | 73.33 |

### Example 3 Determination of thermostability of IPMDH of Sulfolobus sp. and ancestral IPMDH

Because thermostability of *Sulfolobus* sp. IPMDH is very high at pH 7.0, the thermostability thereof at 99°C was determined. In particular, a time required for reducing the activity to 1/2 (half-life T_{1/2}) at 99°C was determined and utilized as the index of the thermostability.

The half-lives of natural and variant (ancestral) enzymes at 99°C were determined as follows: Enzyme solutions having a protein concentration of 0.25 mg/ml (for b', b", b, c and d variants) or 1.0 mg/ml (for abcd variant) were prepared by using a potassium phosphate buffer (20mM KHPO₄, 0.5 mM EDTA, 1 mM DTT, pH 7.0). Also for natural IPMDH, enzyme solutions having protein concentrations of 0.25 mg/ml and 1.0 mg/ml were prepared. These enzyme solutions were heat-treated at 99°C for 10, 20, 30, 60 or 120 minutes. After the completion of the treatment, the enzyme solutions were left to stand in ice for 5 minutes and then centrifuged at 12,000 rpm at 4°C for 20 minutes. The supernatant was recovered from each product. 10 µl of each supernatant was used to determine the activity at 75°C. The determination was repeatedly conducted 3 times for each sample, and the average of results was taken as the residual activity. The residual activity was plotted in a graph wherein the horizontal axis represent the time, and the ordinates represent the relative activity (time 0 was represented as 100). The time at which the relative activity was 50 % was taken as the half-life T_{1/2}. At the same time, the specific activity was also determined. The results are shown in Tables 3 and 4.

**Table 3**

| Half-life and specific activity of natural IPMDH and b', b", b, c and d variants | | |
|---|---|---|
| Type | T_{1/2} (min) | Specific activity (µ/mg) |
| Natural IPMDH of *Sulfolobus* sp. | 10.1 | 11.0 |
| b' variant | 15.8 | 11.0 |
| b" variant | 13.1 | 10.9 |
| b variant | 12.8 | 14.7 |
| c variant | 16.4 | 17.5 |
| d variant | 16.7 | 11.6 |

**Table 4**

| Half-life and specific activity of natural IPMDH and abcd variant | | |
|---|---|---|
| Type | T_{1/2} (min) | Specific activity (u/mg) |
| Natural IPMDH of *Sulfolobus* sp. | 15.3 | 11.0 |
| abcd variant | 23.7 | 11.0 |

It is apparent from these results that the thermostability of all of b', b", b, c, d and abcd variants was improved as compared with that of natural IPMDH. The specific activity of each of b', b" and d variants was also increased.

### Example 4. Construction of ancestral IPMDH from Thermus thermophilus

### (1) Estimation of amino acid sequence of ancestral IPMDH

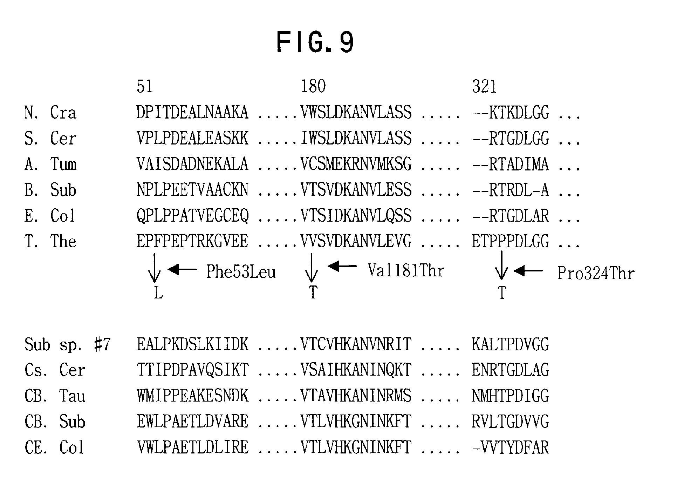

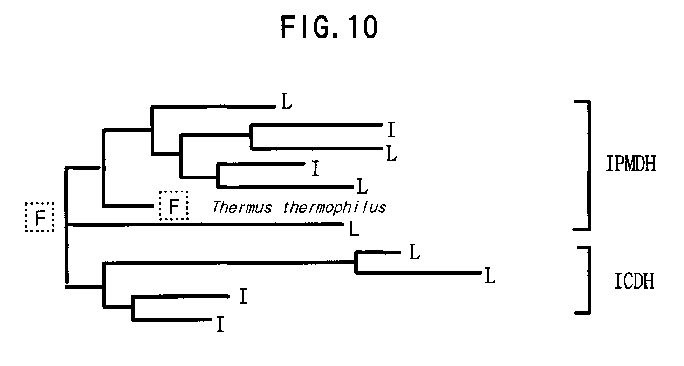

Amino acid sequence of IPMDH and ICDH from representative species which has been cloned were aligned (Fig. 9:Amino acid sequences in Fig. 9 were described in the sequence listing as SEQ ID:57 to SEQ ID:89, from top left to bottom right respectively). Among them, amino acids which are conserved among species and which are different in *Thermus thermophilus* were investigated. Also, considering the information together with the composite phylogenetic tree (Fig.3) of IPMDH and ICDH, the sites were estimated where the tree branches before *Thermus* and the amino acid residue before the branching can be clearly identified. Fig. 10 shows the amino acid residues in various species at the position corresponding to position 53 in *Thermus.* From this, it was clearly suggested that Leu had branched to Phe for *Thermus.* Thus clearly estimated ancestral variants were 3 variants, F53L, V181T and P324T. The meaning of the notation such as F53L, V181T, P324T is identical to the meaning described in Example 1.

### (2) Introduction of mutations

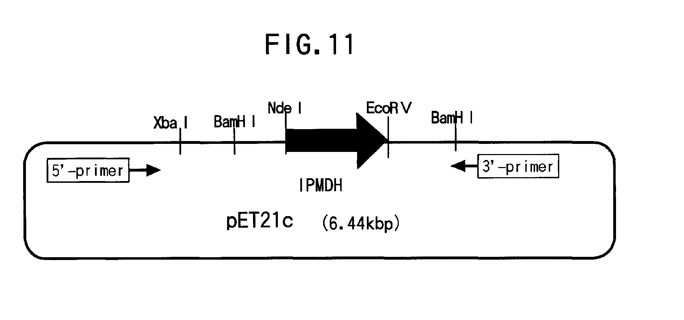

Mutations were introduced in site-specific manner using PCR according to the method of Veronique Picard (Picard, VC. et. al., Nucleic Acid Research, 22, 2587-2591 (1994)). Briefly, the region from 5'-primer to mutant primer was amplified using the plasmid where *Thermus thermophilus* IPMDH (NCBI accession No. AAA16706) was cloned into pET21c (Fig. 11) as a template. Then, full length was amplified by adding 3'-primer. Next, additional 5'-primer was added and the full length was further amplified. P324T could not be amplified using this procedure because the mutation site was located on the 3' end region of IPMDH. Therefore, the reverse oligo 5P324T3 was produced to amplify P324T variant from 3'-end to introduce the mutation. The primers used for mutagenesis were as follows:

5'-primer T7T: : 5'-CTAGTTATTGCTCAGCGGT-3' (SEQ ID:90)
5'-primerT7P : 5'-TAATACGACTCACTATAGGG-3' (SEQ ID:91)
Primer for F53L mutagenesis : 5'-GGGCTCGGGCAAGGGCTCGC-3'
(SEQ ID:92)

Primer for V181T mutagenesis : 5' -AGGTCCGGGTCGGGTCTCC-3'
(SEQ ID:93)

Primer for P324T mutagenesis : 5'-CTTGTCCACGCTCGTCACGTGCTTCCTG3'
(SEQ ID:94)

### Example 5. Comparison between wild type IPMDH from Thermus thermophilus and ancestral IPMDH

### (1) Purification of wild type IPMDH and ancestral IPMDH

Wild type IPMDH from *Thermus thermophilus* and ancestral IPMDH were purified using the similar procedure as described in Example 2, making it a proviso that the third nucleotide of several codons of the gene were changed to A or T to lager production of the protein, because IPMDH gene from *Thermus thermophilus* is GC rich, which may decrease the expression of the gene. The final yields from 1L culture were 184mg/L for wild type, 11.3mg/L for ancestral variant F53L and 8.4mg/L for ancestral variant V181T.

### (2) Determination of thermostability of ancestral IPMDH

Wild type IPMDH and ancestral IPMDH were subjected to heat treatment and the residual activities were determined. For all the experiments, the measurement was conducted three times for each experiment and the residual activity was obtained as the average of the measurements.

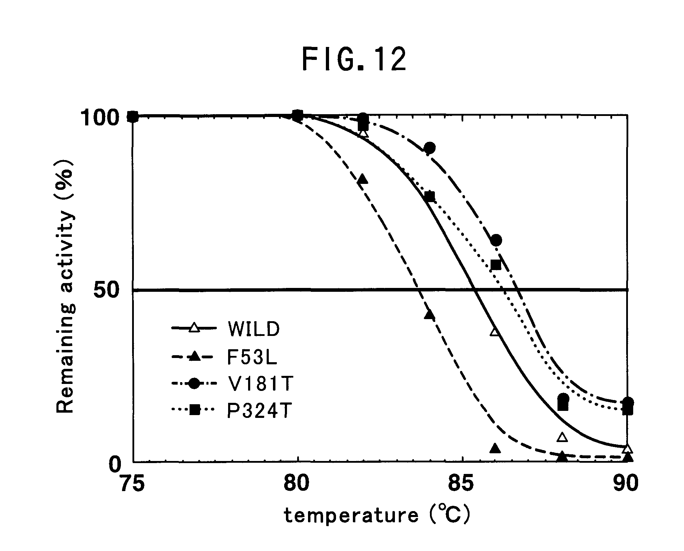

Wild type and ancestral IPMDH protein solution were prepared as a solution of 0.4mg/ml (20mM KHPO₄, pH7.6, 0.5mM EDTA), respectively. 50 *µ*l of each sample was taken in 0.5ml tube and the activity was determined at 50°C after heating at 80, 82, 84, 86, 88, and 90°C for 10 minutes. The temperature was determined where the residual activity reduces to 50%. The results were shown in Fig. 12. The results show that the temperature where the activity reduces to 50% was 85.5°C for wild type, 83.5°C for F53L variant and 86.8°C for V181T variant and 86.5°C for P324T variant. Thus determined temperature was increased by 1.3°C for V181T variant and 1.0°C for P324T variant, although it was decreased by about 2°C for F53L variant.

The time at which the activity reduces to 50% was determined by determining the residual activity at 50°C after the heat treatment for 0, 5, 10, 15 and 20 minutes at 86°C .The results were shown in Table 5.

**Table 5.**

| Time where the residual activity reduces to 50% | | |
|---|---|---|
| | T_{1/2} (min.) | ΔT_{1/2} (min.) |
| Wild Type | 9.4 | |
| F53L | 3.5 | -5.9 |
| V181T | 22.1 | +12.7 |
| P324T | 12.5 | +3.1 |

As can be seen in Table 5, ΔT_{1/2} was increased by 12.7min. for V181T and 3.1min. for P324T, although it was decreased by 5.9min for F53L.

The reason why the thermostability of F53L variant was reduced to less than the thermostability of wild type may reside in the following factors: Investigation of the amino acid sequence around residue 53 revealed that the residue 58 in *Thermus thermophius* is Arg, while it is Leu or Val in many other species. From the fact, it is believed that the structure became unstable by changing the amino acid residue at position 53 to Leu which cannot fill the space between the residue 53 and Arg at position 58, unlike Phe, and the thermostability was reduced as a result.

### (3) CD spectra

Wild type IPMDH and variants F53L, V181T and P324T were prepared as a solution of 0.1mg/ml (20mM KHPO₄, pH7.6), respectively and their secondary structures were investigated using CD (Circular dichroism) spectra ranging 210nm-250nm . NO significant changes were found for each variant compared to wilt type. This indicates that these mutations did not significantly affect the secondary structure of the protein.

### Example 6. Construction of ancestral ICDH from Caldococcus noboribetus

### (1) Estimation of amino acid sequence of ancestral ICDH

Amino acid sequences of IPMDH from representative species and ICDH from various species were obtained from NCBI database and they were subjected to the multiple alignment using Clustal X, an software for alignment (Fig. 14). Also the composite phylogenetic tree was produced using Puzzle, the software for producing a phylogenetic tree, based on these sequences. From the result of alignment and the composite phylogenetic tree, six ancestral mutation, A336F, Y309I, I310L, I321L, A325P and G326S, were predicted using similar procedure as described in Example 1 and 4. The meaning of the notation such as A336F is identical to the meaning described in Example 1 and 4. Among them, since Y309I and I310L, and also A325P and G326S are adjacently located and are located in the same secondary structure, they were considered as a double mutant, respectively. Therefore, Y309/I310L mutation, I312L mutation, A325P/G326S mutation and A336F mutation will be also hereinafter referred to as N1, N2, N3 and N4 mutation, respectively.

### (2) Introduction of mutations

N1, N2, N3 and N4 mutation were introduced by the similar methods in Example 1 and 4 using the plasmid where ICDH from *Caldococcus noboribetus* (NCBI accession No. BAA13177) had been cloned into pET21c, as the template

### Example 7. Comparison between wild type IPMDH from Caldococcus noboribetus and ancestral ICDH

### (1) Purification of wild type ICDH and ancestral ICDH

Wild type ICDH from *Caldococcus noboribetus* and ancestral ICDH were produced in large scale using pET21c and mutant pET21c to which N1-N4 mutation was introduced and *E*. *coli*, as described in Example 2, and then the proteins were purified according to the conventional procedures. The final yields from 1L culture were 10mg/L, 15.4mg/L, 10.9mg/L, 14.2mg/L, 14.2mg/L and 4.39mg/L for wild type, N1 type variant, N2 type variant, N3 type variant and N4 type variant.

### (2) Determination of thermostability of ancestral ICDH

To estimate the thermostability of wild type ICDH from *Caldococcus noboribetus* and each variant, they are subjected to the heat treatment at various temperature (80, 82, 84, 86, 88, 90, 92 and 94°C) for 10 minute, before the residual activity was determined at 70°C. The relationship between the residual activity and temperature was similar to that in Example 5 (see Fig. 12). The temperature where the activity reduces to 50% (T_{1/2}) was 87.5, 88.8, 88.8, 91.3, 74.0°C for wild type, N1-N4 ICDH variants, respectively. The thermostability increased by 1°C for N1 and N2 type ICDH variant and 4°C for N3 type ICDH variant compared to wild type, although the thermostability of N4 type variant was decreased by 13°C.

The specific activity was also determined at 80°C. The relative activities of ICDH variants were about 72, 62, 127 and 21% (based on the activity of wild type as 100%). The specific activities of N1, N2 and N3 type ICDH variants were not significantly changed but the specific activity of N4 type variant of which thermostability had been largely reduced was also significantly decreased.

Since the thermostability of N4 type ICDH variant was significantly reduced, the tertiary structure was additionally investigated. The results showed that Leu327, Tyr363 and Leu364 were located around Ala336 and they formed a hydrophobic pocket. The sites corresponding to Ala336 and Leu327 in other species varied such that they formed a pair in the manner where if one of these residues is a large residue, the other is a smaller residue, such as Phe-Ala, Phe-Gly, Tyr-Ala, Ala-Met. Considering these observations, the reason why the thermostability of N4 type ICDH variant was reduced was believed to be the steric hindrance caused by the alteration from Ala336 to Phe resulted from the compactness of this region.

According to the present invention, the thermostability of protein can be improved by the information of only the primary structure without the information of the secondary and tertiary structures of protein. In particular, the thermostability of thermostable proteins produced by thermophilic bacteria, particularly the thermostable enzymes, can be further improved. When such a thermostable enzyme is used, the reaction can be carried out at a high temperature without temperature control and, therefore, the reaction can be carried out at a high reaction rate at a high temperature. Accordingly, the contamination with unnecessary microorganisms can be minimized.

It is also understood that the examples and embodiments described herein are only for illustrative purpose, and that various modifications will be suggested to those skilled in the art without departing from the spirit and the scope of the invention as hereinafter claimed.

## Claims

1. A method for improving thermostability of proteins, which comprises the steps of
(i) comparing amino acid sequences of proteins from two or more species which evolutionarily correspond to each other in a phylogenetic tree;
(ii) estimating an amino acid sequence of an ancestral protein corresponding to the amino acid sequences compared in step (i); and,
(iii) comparing the amino acid residues in the amino acid sequence in one of the proteins compared in step (i) with amino acid residues at a corresponding position in the ancestral protein estimated in step (ii), and replacing one or more amino acid residues of the protein different from those of the ancestral protein with the same amino acid residues as those of the ancestral protein.

2. The method of claim 1, further comprising the steps of
(iv) testing the proteins obtained in step (iii) for thermostability; and
(v) selecting a protein having improved thermostability.

3. A method for improving thermostability of proteins, which comprises the steps of
(i) comparing amino acid sequences of proteins from two or more species which evolutionarily correspond to each other in a phylogenetic tree by multiple alingment;
(ii) estimating an amino acid sequence of an ancestral protein corresponding to the amino acid sequences compared in step (i); and,
(iii) comparing the amino acid residues in the amino acid sequence in one of the proteins compared in step (i) with amino acid residues at a corresponding position in the ancestral protein estimated in step (ii), and replacing one or more amino acid residues of the protein different from those of the ancestral protein with the same amino acid residues as those of the ancestral protein.

4. The method of claim 3, further comprising the steps of
(iv) testing the proteins obtained in step (iii) for thermostability; and
(v) selecting a protein having improved thermostability.

5. The method for improving thermostability of protein according to claim 1, wherein
(a) thermophilic bacteria or archaebacteria are included in the species from which the protein to be compared is derived in step (i); or
(b) two or more proteins belonging to the same family are included in the proteins to be compared in (i).

6. The method for improving thermostability of protein according to claim 3, wherein
(a) thermophilic bacteria or archaebacteria are included in the species from which the protein to be compared is derived in step (i); or
(b) two or more proteins belonging to the same family are included in the proteins to be compared in (i).

7. A protein improved in thermostability by the method of claim 1.

8. A Nucleic acid encoding the proteins of claim 7.

9. A recombinant DNA molecule containing the nucleic acids of claim 8 in a form being functional for expression.

10. A host cell having the recombinant DNA molecules of claim 9.

11. The method of claim 1, wherein the protein is an 3-isopropylmalate dehydrogenase.

12. The method of claim 1, wherein the protein is an isocitrate dehydrogenase.

13. The method of claim 1, wherein the maximum parsimony method is used for estimating an amino acid sequence of an ancestral protein.

14. The method of claim 3, wherein the maximum parsimony method is used for estimating an amino acid sequence of an ancestral protein.

15. The method of claim 1, wherein the neighbor-joining method is used for estimating an amino acid sequence of an ancestral protein.

16. The method of claim 3, wherein the neighbor-joining method is used for estimating an amino acid sequence of an ancestral protein.
